# EUROPEAN PATENT SPECIFICATION

(11) **EP 2 200 682 B1**
(45) Date of publication and mention of the grant of the patent: **08.02.2012**
(21) Application number: 08805155.2
(22) Date of filing: 08.10.2008
(51) Int. Cl.: A61M 5/32

(54) **HYPODERMIC NEEDLE WITH WATER-SOLUBLE OBSTRUCTION FOR THE ADMINISTRATION OF DRUGS AND VACCINES**
INJEKTIONSNADEL MIT WASSERLÖSLICHEM HINDERNIS ZUR VERABREICHUNG VON ARZNEIMITTELN UND IMPFSTOFFEN
AIGUILLE HYPODERMIQUE COMPORTANT UNE OBSTRUCTION HYDROSOLUBLE POUR L'ADMINISTRATION DE MÉDICAMENTS ET DE VACCINS

(30) Priority: 09.10.2007 ES 200702650
(43) Date of publication of application: 30.06.2010
(73) Proprietor: Azurebio, S.L., 28760 Tres Cantos (Madrid) (ES); Garcia De Castro Andrews, Arcadio, 28240 Hoyo De Manzanares (ES)
(72) Inventor: GARCIA DE CASTRO ANDREWS, Arcadio, E-28240 Hoyo De Manzanares (ES)
(74) Representative: Pons Ariño, Angel
(86) International application number: PCT/EP2008/063494
(87) International publication number: WO 2009/047276

(56) References cited:
- EP-A- 0 060 385
- FR-A- 2 655 859
- US-A- 3 837 284
- US-A1- 2003 225 378
- US-A1- 2004 010 237

## Description

### INVENTION FIELD

The invention refers to hypodermic needles for the parenteral administration of liquids and medicines. The hypodermic needles incorporate a simple addition that ensures that the user cannot expel any amount of the liquid before injection of the needle into the receiving animal or human.

### BACKGROUND

Many drugs are administered parenterally by means of injection by trained medical personnel. The misconception that the air container in the needle can become a problem upon injection has lead to the widespread custom of eliminating the air from the needle by means of a gentle pressure over the syringe until a small amount of liquid is expelled through the sharp end of the needle. This does not generally represent a problem in the administration of large volumes where the potential loss represents a small fraction of the total volume of liquid to be injected. However, this practice can become a serious limitation in the injection of small volumes of liquids or in situations in which the first fractions of liquid to be injected contains a large fraction of the dose of the drug to be administered.

Un example of preloaded devices that incorporate small volumes for the low cost administration of medicines is exemplified by Uniject^{™}. Devices of this kind have also been proposed for the administration of formulations that do not require refrigeration. Examples of these formulations are described in patent application WO-98/41188 that incorporates solid suspensions in injectable oils, and also in patent application WO-02/032402 that incorporates fluorocarbons as a means to suspend solid soluble particles containing a drug or vaccine. However, in these preloaded devices incorporating small volumes, the common practice of expelling the air contained in the needle can result in the loss of a considerable fraction of the dose to be administered.

Another method proposed in the administration of drugs and vaccines incorporates the stabilisation of drugs in a solid form in a cartridge located at the syringe end of the hypodermic needle. Devices of this kind are an object of an innovation program promoted by the World Health Organisation [Lloyd J. (2000) Technologies for Vaccine Delivery in the 21st Century. WHO/V&B/00.35]. Examples of these devices are described in patent application US200310068354 that incorporates a membrane on which the DNA material which constitutes the vaccine is lyophilised, and in patent application WO2007/057717 which incorporates a cartridge holding a fibrillar support with a large surface area on which the drug or vaccine is stabilised within a fine film of dry amorphous glass.

In both cases, the injection of an aqueous solution through the cartridge results in the rapid dissolution and carry over of the medicament, most of it in the first fraction of the liquid. Once more, the ejection of a small amount of liquid prior to injection can result in the administration of a suboptimal dose of the drug or vaccine.

US 2004/010237 A discloses a needle comprising a rounded end provided with a sharp tip whereby said tip is made of a material soluble in body fluids.

In general, the existing devices for the parenteral administration of liquids do not provide a method to avoid the user in expelling a small amount of liquid through the injection needle prior to the injection, and this can originate a considerable reduction of the dose to be administered.

It would therefore be desirable to have simple means in the parenteral administration of liquids that prevents the end user from expelling a fraction of the medicament prior to injection. This would be of particular interest in the administration of non-aqueous suspensions of medicines, or in the administration of medicines which are stabilised within a cartridge at the syringe-end of the needle, were the initial disposal of a small volume may carry-over a considerable proportion of the total dose to be administered.

### INVENTION SUMMARY

The common custom of sanitary personnel to expel an amount of liquid before proceeding to the injection of a drug or medicament can result in the administration of a fraction of the recommended dose. This is particularly relevant in the parenteral administration of small volumes, in preloaded devices, or in devices that incorporate the drug or vaccine in a cartridge in-line within the syringe-end of the needle. The incorporation of a soluble obstruction within the sharp-end of the hypodermic needle provides needles that are only operational once they are injected and the soluble obstruction dissolves in the tissues of the injected human or animal subject. These needles avoid the possibility that the user can expel part of the dose prior to injection and facilitate the administration of the full dose of the drug or vaccine.

### DESCRIPTION OF THE FIGURES

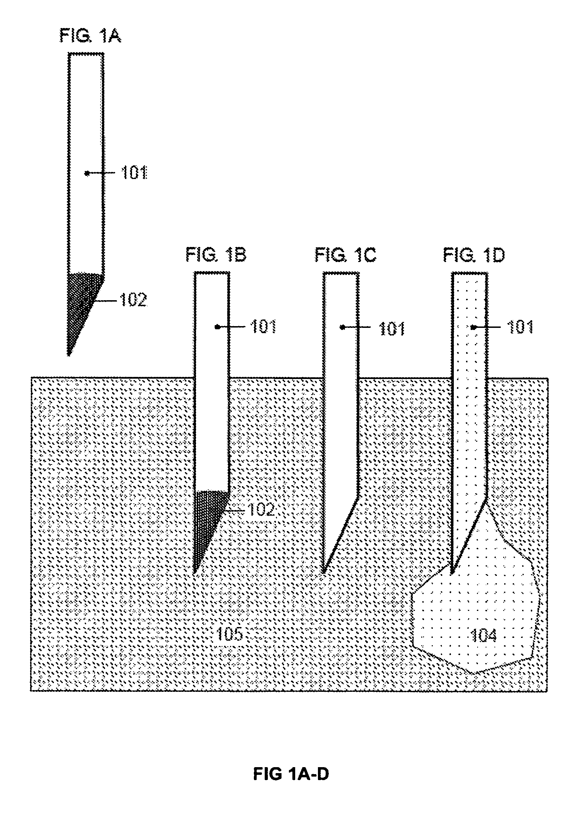

**Figures 1A-D.** **Represent the steps in the process of injection of a hypodermic needle which contains a soluble obstruction at its sharp-end.** Figures 1A-D represent longitudinal sections of the different moments in the injection process of a hypodermic needle 101 that incorporates in its sharp-end an obstruction 102 with a soluble material. Figure 1A represents a hypodermic needle prior being used. Figure 1B represents a hypodermic needle at the moment of injection in the human or animal tissues 105. Figure 1C represents a hypodermic needle at the moment immediately alter injection once the obstruction 102 has dissolved trough contact with the surrounding tissues. Figure 1D represents the hypodermic needle at the moment in which the drug or vaccine in liquid solution or suspension 104 is administered.

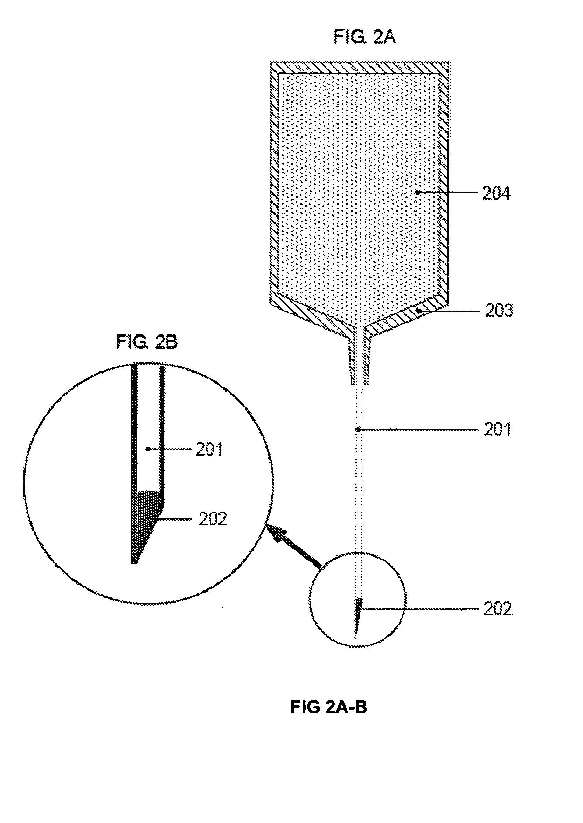

**Figures 2A-B.** **Represent a preloaded device loaded with a drug or vaccine in a liquid format and incorporating a soluble obstruction at the sharp-end of the hypodermic needle.** Figure 2A represents a longitudinal section of an injector device 203 for drugs and vaccines in a liquid solution or suspension 204 that incorporate a soluble obstruction 202 at the sharp-end of a hypodermic needle 201. Figure 2B represents an amplified view of the sharp-end of the hypodermic needle 201 and the soluble obstruction 202.

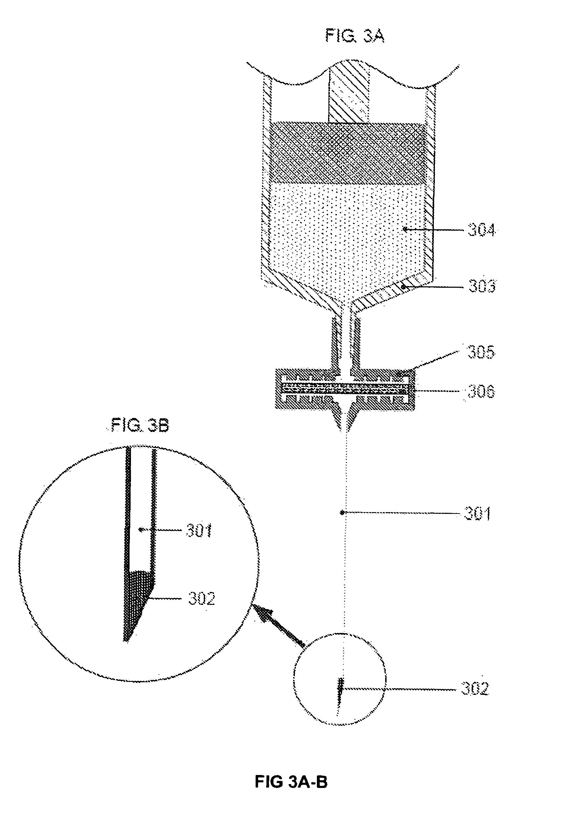

**Figures 3A-B.** **Represent a device containing a drug or vaccine in a solid format within a cartridge of a hypodermic needle and containing a soluble obstruction at the sharp-end of the hypodermic needle.**
   Figure 3A represents a longitudinal section of a device that at the sharp-end of a hypodermic needle 301 incorporates a soluble obstruction 302. The drug 306 in a solid format is incorporated within a cartridge 305 located at the syringe end of the hypodermic needle 301. The injection device 303 contains water for injection 304. Figure 3B represents an amplified view of the sharp-end of the hypodermic needle 301 and the soluble obstruction 302.

### DETAILED DESCRIPTION OF THE INVENTION

The present invention refers to hypodermic needles 101, 201 or 301 for the administration of solutions and suspensions of drugs, medicines and vaccines, that contain at their sharp-end a water-soluble obstruction 102, 202 or 302, as is represented in Figures 1A-D, 2A-B and 3A-B.

Penetration of the hypodermic needles 101, 201 or 301 in living tissue of a human or animal subject results in the almost instantaneous dissolution of the obstruction 102, 202 or 203, and permits the injection of the liquid containing the drug or vaccine. The incorporation of the obstruction 102, 202 or 302 avoids the user from expelling part of the liquid contained within the injection device prior to the needle penetrating the tissues of the receiving animal or human subject. The hypodermic needles 101, 201 or 301 have preferred internal diameters ranking from 0.1 mm y 1mm, and the capacity of administering drugs and vaccines by injection to depths up and over 5 cm. However it is acknowledged that larger internal diameter needles may be applicable in other fields of animal and plant health. The obstruction 102, 202 or 302 is preferably composed by materials which are rapidly soluble in aqueous media and that dissolve once the needle penetrates living tissues. Examples of these include, with out limitation, carbohydrates, sugar alcohols, amino acids, proteins, polymers, salts and/or mixtures thereof. Applicable carbohydrates and sugar alcohols include, with out limitation, maltodextrin, trehalose, raffinose, cellobiose, melezitose, glucose, fructose, maltulose, iso-maltulose, lactulose, maltose, gentobiose, lactose, galactose, isomaltose, manose, maltitol, lactitol, eritrol, palatinitol, inositol, xilitol, mannitol, sorbitol, dulcitol and/or ribitol and mixtures thereof. Preferably the carbohydrates are chosen from those that are acceptable for injection, with great solubility, and solid at a temperature of at least 45°C, like for example mannitol, trehalose, raffinose, sucrose and/or glucose. Preferably the amino acids are chosen among those that are acceptable for injection, highly soluble and solid at a temperature up to 45°C like for example proline, cysteine, arginine, glutamine and/or glycine, and mixtures thereof. Applicable proteins include, with out limitation, collagen, hyaluronic acid, fibronectin, gelatine, and/or agaroses, and mixtures thereof. The applicable polymers, include with out limitation, polyvinyl alcohols, polyvinylpyrrolidone and/or polyethylene glycols, and mixtures thereof. Compatible solutes such as, with out limitation, glycine-betaine, ectoin, hydroxyectoin, can also be incorporated. Moreover, the obstruction 102, 202 or 302 can incorporate materials that result in an effervescent reaction when in contact with an aqueous media. Examples of this, with out limitation, include sodium carbonates. Other acceptable salts which are rapidly soluble and have the additional benefit of being efflorescent include, sodium sulphate, acetate trihydrate, tetraborate decahydrate (Borax), bromoiridite dodecahydrate, carbonate heptahydrate metaperiodate trihydrate, metaphosphate hexahydrate, hydrogen orthophosphate dodecahydrate, sulphite heptahydrate, thiosulphate pentahydrate, calcium lactate, magnesium salicylate tetrahydrate, magnesium sulphate heptahydrate, and ammonium sulphate.

The incorporation of the obstruction 102, 202 or 302 in the hypodermic needle can be done by means of the incorporation of solutions of the soluble material and subsequent solidification by evaporation. The obstruction can also be incorporated by heating of the solid obstruction materials at temperatures in which they become malleable or liquid. The incorporation of a predetermined volume and subsequent cooling results in the fabrication of the desired soluble obstruction. Other methods that permit the incorporation of a soluble obstruction include the incorporation of soluble membranes or puncture with the needle of a solid matrix that becomes incorporated at the needle end.

The present invention is applicable, among others, to preloaded devices such as those represented in Figure 2A-B. The incorporation of the obstruction 202 ensures that administration of the drug or vaccine 2004 contained in the reservoir 203 is only possible once the hypodermic needle has been injected and the obstruction 202 has dissolved in the receiving living tissues. One preferred realisation of the present invention incorporates devices preloaded with solutions or suspensions of the drug or vaccine in non aqueous liquids, such as, and with out limitation, oils or fluorocarbons. Preferably the material that composes the obstruction 202 is not soluble in these non-aqueous liquids and therefore does not dissolve until the needle has penetrated the receiving living tissue.

In another realisation represented in Figures 3A-B, the hypodermic needle 301 that incorporates at its sharp-end the obstruction 302, also incorporates a cartridge 305 that contains the drug or vaccine 306 stabilised in a solid format. Administration of the drug or vaccine involves the assembly with an injector device 303 containing saline or water for injection 304. Injection of the hypodermic needle into living tissues results in the dissolution of the obstruction 302 permitting the flow of the water for injection 304 through the cartridge, and carry over of the dissolved drug or vaccine 306.

The hypodermic needles describes in the present invention incorporate at their sharp-end a soluble obstruction 102, 202 or 302 which makes them functional only once they encounter an aqueous media such as a living tissue, as is illustrated, with out limitation, in the following three examples.

### Example 1: Injection of tetanus vaccine formulated as suspension in oil by means of a preloaded device incorporating a hypodermic needle with a soluble obstruction at its sharp-end.

The device represented in Figures 2A-B was made to incorporate a soluble obstruction 202 of trehalose at the sharp-end of a 0.6 mm internal diameter stainless steel bevelled hypodermic needle 201. For this, the sharp-end of the needle was dipped in a trehalose melt at about 100°C. Cooling of the tip resulted in the formation of an obstruction 202 of solid amorphous trehalose glass. The needle incorporating the obstruction was assembled to a flexible PVC ampoule as injector device 203. As a model drug or vaccine 204, the ampoule contained 1 ml of a suspension in sesame seed oil of 1-10 µm trehalose particles containing stabilised tetanus vaccine. Pressure of the final user on the pre-filled ampoule did not result in the ejection of any amount of the contained suspension. Intramuscular injection of the needle in a guinea pig resulted in the immediate dissolution of the obstruction 202 and permitted the administration of the complete dose of the vaccine suspension by gentle pressure by the user over the ampoule.

### Example 2: Injection of a hepatitis B vaccine stabilised in a solid format in a cartridge located at the syringe end of a hypodermic needle incorporating a soluble obstruction at its sharp-end.

The device represented in Figures 3A-B was manufactured to incorporate a soluble obstruction 303 of mannitol at the sharp end of a 0.3 mm internal diameter stainless steel bevelled hypodermic needle 201. For this the sharp-end tip of the hypodermic needle was introduced in a 60% mannitol solution in water at about 70°C. Removal of the needle and cooling of the tip resulted in the formation of a solid obstruction with a mannitol crystal at the sharp-end of the needle. The needle carried at the syringe end a cartridge 305 containing as a model drug or vaccine 306 a hepatitis B vaccine stabilised in an amorphous water-soluble trehalose glass. Prior to injection a conventional syringe 303 containing 1 ml of water for injection, was assembled to the hypodermic needle 301. Once assembled, pressure over the syringe 303 did not result in the ejection of any amount of liquid trough the syringe. Subcutaneous injection of the needle into a guinea pig and gentle pressure over the syringe resulted in the immediate dissolution of the obstruction 302 permitting the flow of water for injection 304 through the cartridge 305 and administration of a full dose of the drug 306.

### Example 3: Evaluation of suitability of different needle obstruction materials.

A hypodermic bevelled stainless steel needle, as for example in 101, with an internal diameter of 0.8 mm was used to evaluate suitability of different materials to create the sharp-end obstruction 102. The needles containing approximately 1-3 mm³ volume of the different obstruction materials were assembled on to a syringe containing 1 ml sesame seed oil and injected into a set 3% gelatine block at 25°C as a model of a living tissue 103. Dissolution time was estimated from the time of injection. Rapidly dissolving materials generally categorised as suitable needle-end obstruction materials (++++) while slower dissolving materials generally resulted in delayed injection and were categorised as less suitable obstructing materials (+).

| **Obstruction Material** | **Suitability as needle obstructing material** |
|---|---|
| Trehalose (from melt) | +++ |
| Trehalose + 2% sodium bicarbonate (from melt) | ++++ |
| Trehalose + 5% Calcium Lactate + 2% sodium bicarbonate | ++++ |
| Sucrose (from solution) | +++ |
| Sucrose + 5% Sodium Sulphate (from solution) | ++++ |
| Trehalose + 5% Calcium Lactate (from solution) | +++ |
| 20% Mannitol + 70% Trehalose (from melt) | ++++ |
| Mannitol (from melt) | ++++ |
| Hydroxyectoine (from solution) | ++++ |
| Glycine Betaine (from solution) | ++++ |
| Polyvinyl alcohol (from solution) | + |
| Proline | ++++ |
| Arginine·HCl | ++++ |
| Cysteine | + |
| Alanine | ++++ |
| Tyrosine | + |

## Claims

1. Hypodermic needles (101, 201, 301) incorporating at their sharp-end a water-soluble obstruction (102, 202 or 302) that dissolves once the hypodermic needle is injected into human or animal tissues to allow passage of the liquid to be injected.

2. Hypodermic needles according to Claim 1 in which the obstruction include maltodextrin, trehalosa, raffinose cellobiose, melezitose, glucose, fructose, sucrose, maltulose, iso-maltulose, lactulose, maltose, gentobiose lactose, galactose, isomaltose, manose, maltitol, lactitol, erythritol, palatinitol, inositol, xilitol, mannitol, sorbitol, dulcitol and/or ribitol.

3. Hypodermic needles according to Claim 2 in which the agents included in the obstruction are selected among trehalose, raffinose, sucrose, glucose and/or mannitol.

4. Hypodermic needles according to any of Claims 1 to 3 in which in which the obstruction incorporates proline, cisteine, arginine, glutamine, glycine, glycine-betaine, ectoin and/or hydroxyectoin.

5. Hypodermic needles according to any of Claims 1 to 4 in which in which the obstruction incorporates collagen, hyaluronic acid, fibronectin, gelatine, and/or agarose.

6. Hypodermic needles according to any of Claims 1 to 5 in which in which the obstruction incorporates polyvinyl alcohols, polyvinylpyrrolidone and/or polyethylene glycols.

7. Hypodermic needles according to any of preceding Claims 1 to 6 in which the obstruction incorporates agents that promote an effervescence upon injection.

8. Hypodermic needles according to Claim 7 in which the agent that causes effervescence upon injection is a sodium carbonate.

9. Hypodermic needles according to Claims 1 to 8 which incorporates a rapidly dissolving efflorescent salt.

10. Hypodermic needles according to Claim 9 in which the salt is sodium sulphate, acetate trihydrate, tetraborate decahydrate, bromoiridite dodecahydrate, carbonate heptahydrate metaperiodate trihydrate, metaphosphate hexahydrate, hydrogen orthophosphate dodecahydrate, sulphite heptahydrate, thiosulphate pentahydrate, calcium lactate, magnesium salicylate tetrahydrate, magnesium sulphate heptahydrate, and/or ammonium sulphate.

11. Incorporation of the hypodermic needles (101, 201, 301) according to any of Claims 1 to 10, in devices designed for the parenteral administrations of drugs and vaccines, and/or products in animal or human health.

12. Incorporation of the hypodermic needles (101, 201) according to Claim 11 in devices in which the drugs or vaccines are formulated into non-aqueous liquids.

13. Incorporation of the hypodermic needles (101, 301) according to Claim 11 in devices in which the drugs or vaccines are contained in a solid format in a cartridge, filter or support (305).

## Patentansprüche

1. Hypodermische Nadeln (101, 201, 301), die an ihrem scharfen Ende eine wasserlösliche Abdichtung (102, 202 oder 302) umfassen, die sich auflöst, nachdem die hypodermische Nadel in menschliches oder tierisches Gewebe injiziert wurde, um so den Durchfluss der zu injizierenden Flüssigkeit zu ermöglichen.

2. Hypodermische Nadeln nach Anspruch 1, bei denen die Abdichtung Maltodextrin, Trehalose, Raffinose Cellobiose, Melezitose, Glukose, Fruktose, Saccharose, Maltulose, Iso-Maltulose, Lactulose, Maltose, Gentobiose-Laktose, Galaktose, Isomaltose, Manose, Maltitol, Lactitol, Erythritol, Palatinitol, Inositol, Xilitol, Mannit, Sorbitol, Dulcitol und/oder Ribitol umfasst.

3. Hypodermische Nadeln nach Anspruch 2, bei denen die Wirkstoffe in der Abdichtung aus Trehalose, Raffinose, Saccharose, Glukose und/oder Mannitol ausgewählt sind.

4. Hypodermische Nadeln nach einem der Ansprüche 1 bis 3, bei denen die Abdichtung Prolin, Cystein, Arginin, Glutamin, Glycin, Glycin-Betain, Ectoin und/oder Hydroxyectoin umfasst.

5. Hypodermische Nadeln nach einem der Ansprüche 1 bis 4, bei denen die Abdichtung Kollagen, Hyaluronsäure, Fibronektin, Gelatine und/oder Agarose umfasst.

6. Hypodermische Nadeln nach einem der Ansprüche 1 bis 5, bei denen die Abdichtung Polyvinylalkohole, Polyvinylpolypyrrolidon und/oder Polyethylenglykole umfasst.

7. Hypodermische Nadeln nach einem der Ansprüche 1 bis 6, bei denen die Abdichtung Wirkstoffe umfasst, die nach der Injektion ein Schäumen fördern.

8. Hypodermische Nadeln nach Anspruch 7, bei denen der Wirkstoff, der nach der Injektion das Schäumen bewirkt, ein Natriumcarbonat ist.

9. Hypodermische Nadeln nach einem der Ansprüche 1 bis 8, die ein schnell auflösendes schäumendes Salz umfassen.

10. Hypodermische Nadeln nach Anspruch 9, bei denen das Salz Natriumsulfat, Acetat-Trihydrat, Tetraborat-Decahydrat, Bromoiridit-Dodecahydrat, Carbonat-Heptahydrat-Metaperiodat-Trihydrat, Metaphosphat-Hexahydrat, Wasserstoff-Orthophosphat-Dodecahydrat, Sulfit-Heptahydrat, Thiosulphat-Pentahydrat, Kalziumlaktat, Magnesium-Salicylat-Tetrahydrat, Magnesium-Sulfat-Heptahydrat und/oder Ammoniumsulfat ist.

11. Einfügung von hypodermischen Nadeln (101, 201, 301) nach einem der Ansprüche 1 bis 10, in Geräte, die zur Verabreichung von Arzneimitteln und Impfstoffen und/oder Produkten für die tierische oder menschliche Gesundheit konzipiert sind.

12. Einfügung von hypodermischen Nadeln (101, 201) nach Anspruch 11, in Geräte, bei denen die Arzneimittel oder Impfstoffe in nicht wässrigen Flüssigkeiten formuliert sind.

13. Einfügung von hypodermischen Nadeln (101, 301) nach Anspruch 11, in Geräte, bei denen die Arzneimittel oder Impfstoffe in einem festen Format in einer Kartusche, einem Filter oder auf einem Träger (305) enthalten sind.

## Revendications

1. Aiguilles hypodermiques (101, 201, 301) comportant à leur extrémité pointue une obstruction soluble dans l'eau (102, 202 ou 302) qui se dissout dès que l'aiguille hypodermique est piquée dans des tissus humains ou animaux afin de permettre le passage du liquide devant être injecté.

2. Aiguilles hypodermiques selon la revendication 1 dans lesquelles l'obstruction comprend de la maltodextrine, du tréhalose, raffinose, cellobiose, mélézitose, glucose, fructose, saccharose, maltulose, isomaltulose, lactulose, maltose, gentiobiose, lactose, galactose, isomaltose, mannose, maltitol, lactitol, érythritol, palatinitol, inositol, xylitol, mannitol, sorbitol, dulcitol, et/ou du ribitol.

3. Aiguilles hypodermiques selon la revendication 2 dans lesquelles les agents inclus dans l'obstruction sont sélectionnés parmi le tréhalose, raffinose, saccharose, glucose et /ou le mannitol.

4. Aiguilles hypodermiques selon n'importe laquelle des revendications 1 à 3 dans lesquelles l'obstruction comprend de la proline, cystéine, arginine, glutamine, glycine, glycine-bétaïne, ectoïne, et/ou de l'hydroxyectoïne.

5. Aiguilles hypodermiques selon n'importe laquelle des revendications 1 à 4 dans lesquelles l'obstruction comprend du collagène, de l'acide hyaluronique, de la fibronectine, gélatine et/ou de l'agarose.

6. Aiguilles hypodermiques selon n'importe laquelle des revendications 1 à 5 dans lesquelles l'obstruction comprend des alcools polyvinyliques, du polyvinylpyrrolidone et/ou des polyéthylèneglycols.

7. Aiguilles hypodermiques selon n'importe laquelle des revendications précédentes 1 à 6 dans lesquelles l'obstruction comprend des agents qui favorisent une effervescence après l'injection.

8. Aiguilles hypodermiques selon la revendication 7 dans lesquelles l'agent qui provoque l'effervescence après l'injection est un carbonate de sodium.

9. Aiguilles hypodermiques selon les revendications 1 à 8 qui comprennent un sel efflorescent à dissolution rapide.

10. Aiguilles hypodermiques selon la revendication 9 dans lesquelles le sel est du sulfate de sodium, de l'acétate trihydraté, du tétraborate décahydraté, bromoiridite dodécahydraté, carbonate heptahydraté, métapériodate trihydraté, métaphosphate hexahydraté, de l'orthophosphate d'hydrogène dodécahydraté, du sulfite heptahydraté, thiosulfate pentahydraté, lactate de calcium, salicylate de magnésium tétrahydraté, sulfate de magnésium heptahydraté, et/ou du sulfate d'ammonium.

11. Intégration des aiguilles hypodermiques (101, 201, 301) selon n'importe laquelle des revendications 1 à 10, dans des dispositifs conçus pour les administrations parentérales de médicaments et de vaccins, et/ou de produits de santé animale ou humaine.

12. Intégration des aiguilles hypodermiques (101, 201) selon la revendication 11, dans des dispositifs dans lesquels les médicaments ou vaccins sont formulés dans des liquides non aqueux.

13. Intégration des aiguilles hypodermiques (101, 301) selon la revendication 11, dans des dispositifs dans lesquels les médicaments ou vaccins sont contenus sous une forme solide dans une cartouche, un filtre ou un support (305).
